Europäisches Patentamt

European Patent Office

Office européen des brevets

(19)

(11) Publication number: **0 303 287 B1**

(12) **EUROPEAN PATENT SPECIFICATION**

(45) Date of publication of patent specification: 30.10.91    (51) Int. Cl.⁵: **A61F 2/36**

(21) Application number: **88113158.5**

(22) Date of filing: **12.08.88**

(54) Hip-joint endoprothesis.

(30) Priority: **13.08.87 PL 267363**

(43) Date of publication of application:
    **15.02.89 Bulletin 89/07**

(45) Publication of the grant of the patent:
    **30.10.91 Bulletin 91/44**

(84) Designated Contracting States:
    **AT BE CH DE ES FR GB GR IT LI LU NL SE**

(56) References cited:
    **EP-A- 0 146 192**
    **EP-A- 0 170 982**

(73) Proprietor: **Huta Baildon**
    **ul. Zelazna 9**
    **PL-40-952 Katowice(PL)**

(72) Inventor: **Lejawka, Jerzy, M. Sc. Mechn. Eng.**
    **ul. Piastow 16/121**
    **40-868 Katowice(PL)**
    Inventor: **Tuziemski, Aleksander**
    **ul. Gospodarcza 18**
    **41-200 Sosnowiec(PL)**
    Inventor: **Christoph, Henryk**
    **ul. Dzierzynskiego 70**
    **40-121 Katowice(PL)**
    Inventor: **Tylman, Donat, Prof., Dr. Med., Sur-**
    **geon**
    **ul. Nowiniarska 1/14**
    **00-235 Warszawa(PL)**
    Inventor: **Wasik, Ryszard, Dr. Med., Surgeon**
    **ul. Stalmacha 26/6**
    **40-058 Katowice(PL)**
    Inventor: **Koszkul, J zef**
    **Al. Wyzwolenia 2/28**
    **42-200 Czestochowa(PL)**
    Inventor: **Stodolnik, Bogdan, Dr. Mech. Eng.**
    **ul. Ok lna 133/69**
    **42-200 Czestochowa(PL)**
    Inventor: **Raczka, Krzysztof**
    **ul. Tysiaclecia 16/172**
    **40-868 Katowice(PL)**
    Inventor: **Tuszynski, Wieslaw, Dr. Med. Sur-**
    **geon**
    **ul. Meander 18/3**
    **02-791 Warszawa(PL)**

(74) Representative: **Füchsle, Klaus, Dipl.-Ing. et al**
    **Hoffmann . Eitle & Partner Patentanwälte Ar-**
    **abellastrasse 4**
    **W-8000 München 81(DE)**

## Description

The invention relates to a hip-joint endoprothesis according to the features of the preamble of claim 1. Such an endoprothesis is known from EP-A-0 146 192.

The object of this invention is a hip-joint endoprothesis combining a housing and a tubular joining element.

There are various types of endoprothesis currently known:

a. Endoprothesis with ball mounted on a cushion and fixed onto a brace.
b. Self-implanting endoprothesis.
c. Needle-type elastic endoprothesis,
d. Elastic brace-and-casing endoprothesis
e. Cone brace-and-casing endoprothesis.

PL-A-111200 and PL-A-130941 as well as DE-A-2 618 763, DE-A-2 142 820 and DE-A-2 641 770 refer to a hip-joint endoprothesis with a ball mounted on a cushion and fixed in the upper part of a brace.

The invention disclosed in PL-A-111200 consists of an endoprothesis where the ball is mounted on a neck and separated from the brace by a plastic spacer ring washer acting as thrust bearing for the ball rotating around the metal neck.

The endoprothesis as shown in PL-A-130941 is made out of plastics and has a ball mounted on a frustum-shaped washer with a cylindrical port.

There are also endoprothesis with a cushion made out of high density polyethylene. This cushion can be placed either between the ball and brace's neck allowing some ball rotation around the neck (DE-A-2 641 820) or for shock absorption between the endoprothesis and its mounting (DE-A-2 641 770).

DE-A-2 681 763 concerns an endoprothesis where the brace is ended by a neck receiving a ball with a spacer correction washer made out of precious metal, allowing regulation of the ball's mounting height and correcting inaccuracies on the joint's surface.

The above mentioned endoprotheses have a number of disadvantages. The flange between the brace and the ball of the endoprothesis inserted into thigh-bone cavity rests directly on the bone thus bearing on Adam's bow, what leads to its degradation. Bone cement is used to immobilize the endoprothesis' curved brace in a drilled bone opening what makes the intervention difficult since it has to be introduced rapidly and correctly. Moreover, the bone cement shrinks as it polymerizes and is toxical at the first phase of setting, causing thermic superficial necrosis of osseous tissue. It also looses its mechanical properties with age. For these reasons the durability of mounting is limited to several years only and a new intervention is required. Following micro-movements the implant becomes loose and therefore the endoprothesis has to be removed.

Similarly, the use of a self-implanting endoprothesis can also lead to unsatisfactory results. There are numerous cavings, pores and ports on the brace's surface facilitating osseous tissue growth throughout them, and leading to an improved stabilization of the endoprothesis.

DE-A-33 104 86 describes a self-implanting endoprothesis which has a stem with individually shaped rings all around, matching with a relevant bone cavity.

This kind of endoprothesis can intergrate well with the bone. A satisfactory force transmission depends upon the tissue growth around the rings. When it is not sufficient, some local stress concentration can occur and a bending moment damages the bone. The endoprothesis is thus exposed to crack risk.

The main disadvantage of this kind of endoprothesis is a slow growth of osseous tissue around the stem and the necessity for the hip-joint not to be exposed to loads for a certain time. Therefore, a self-implanting endoprothesis applies essentially to young patients. The use of a rigid endoprothesis for active patients where greater loads are involved usually leads to fatique cracks. Thus the tenacity of endoprothesis-bone functional combination is wakened what results in a stem migration within the bone cavity. In such a case another operation is inevitable.

There are also needle-shape endoprothesis aiming at force flow transmission from the hip-bone to a resect part of the thigh-bone through the ball. They are also supposed stabilize the brace in certain areas inside the cavity and eventually to improve tenacity on a greater scale.

As practical experience has shown, an elastic needle-type brace is not well performing since it does not assure proper interaction between the endoprothesis and the thigh-bone, due to a specific force distribution and transmission.

Another type is a complex elastic hip endoprothesis interacting with a resect thigh-bone. It consists of an elastic armoured core and a cone casing made out of a plastic compound slightly thicker than the inner diameter of the stem canal. When the endoprothesis' brace is introduced into the properly shaped tubular canal, the plastic casing is compressed and a stress occurs due to the wedge-effect of the brace. Besides, the flange under the ball rests on the thigh-bone.

A similar solution is applied to the intramarrow endoprothesis described in DE-A-33 113 26 which on one hand is incorporated into the hip-joint and on the other hand is equipped with a stem generally matching with the stem canal where it is to be inserted. The stem is coated with a porous elastic material slightly exceeding the diameter of the stem canal. When it is inserted into the bone marrow cavity, its coating is compressed and the flange between the stem and the hip-joint rests on Adam's bow.

The flange adversely affects Adam's bow, causing its degradation. Moreover, when a diameter clearance occurs due to improperly prepared stem canal or due to considerable time of using endoprothesis, the flange makes it impossible to insert the stem deeper into the canal and thus to compensate the clearance between the elastic coating and the bone. The endoprothesis eventually begins moving inside the bone. The flat shape of the stem makes it difficult to prepare an accurate stem canal in the bone in order to obtain a solid mounting of the endoprothesis.

Another solution is applied to an endoprothesis composed of a housing and a partitioned casing. A ball with a flange and a cone brace constitute the housing. The casing consists of two or more oblong cone elements. The brace and the casing are both curved. First, the casing elements are installed in the resect cavity. Then the stem is inserted carefully so that the ball's flange rests on the casing's edge. Acting as a wedge, the stem widens the casing spreading its parts radially and thus the endoprothesis is mounted in the cavity.

The common characteristic and disadvantage of all above mentioned systems of mounting an endoprothesis with the use of a plastic joining element is the lack of uniform contact of the fixing surfaces of the joining elements all around the bone marrow cavity, avoiding considerable stress variations on the osseous tissue which are biologically harmful. The main cause is an uneven inner surface of the bone. Therefore, the bone has to be drilled in order to obtain a slightly conic shape what additionally weakens the bone. Moreover, every endoprothesis with a joining element has a flange which rests on the resect neck thigh-bone, causing its degradation.

The invention intends to develop a hip-joint endoprothesis, the construction of which would:

a. facilitate a smooth transmission of the force flow from the implant's stem to the resect bones,

b. create a radial stress preventing it from moving inside the bone cavity,

c. assure a steady force transmission from the stem to the resect bone,

d. exclude the use of a bone cement being toxic to human organism,

e. prevent it from sliding deeper into the stem canal.

This object is achieved by a hip-joint endoprothesis as defined in the claims.

The hip-joint endoprothesis according to the invention is composed of a housing in the form of a straight symmetry axis frustum-shaped brace, equipped with a ball on a pivot and a wedge-shaped locking lip below the ball and also a joining element, having an inner straight-cut cone surface and a lateral orifice in its upper part, matching with the wedge-shaped locking lip of the housing.

This solution has a number of advantages. All known endoprotheses have a flange necessary to position them in the bone marrow cavity, however, pressing down on the thigh-bone's neck and causing its degradation. In contrast, the proposed solution avoids a flange on both housing and joining element, thus facilitating the most suitable installation of the endoprothesis in a resect bone from the biological viewpoint. The straight cone stem of the housing can be inserted in the joining element in a way which eliminates a possible clearance between the endoprothesis and the bone marrow cavity. Thus, no migration of endoprothesis inside the bone, leading to its damage, can occur. This mounting of endoprothesis grants a proper and even force transmission from the stem to the resect bone. The wedge-shaped locking lip on the endoprothesis' housing, inserted into the orifice in the joining element, prevents the housing from moving inside the joining element. The outer cylindrical surface of the joining element secures the endoprothesis from sliding deeper into the stem canal. An additional substantial advantage of this endoprothesis is the lack of a harmful toxic bone cement.

The invention will now be explained with reference to the attached drawings.

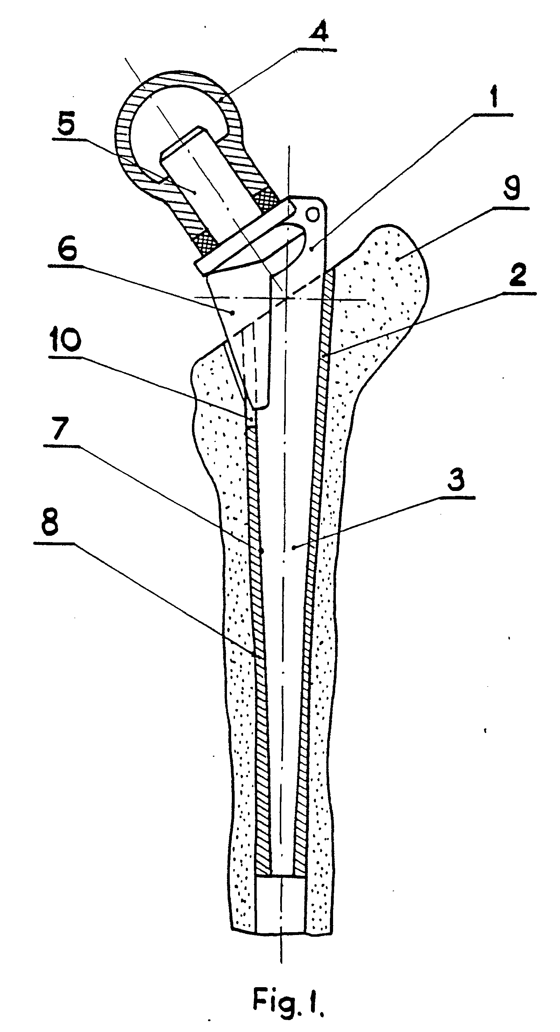

Fig. 1     shows a cross section of endoprothesis mounted in a bone;

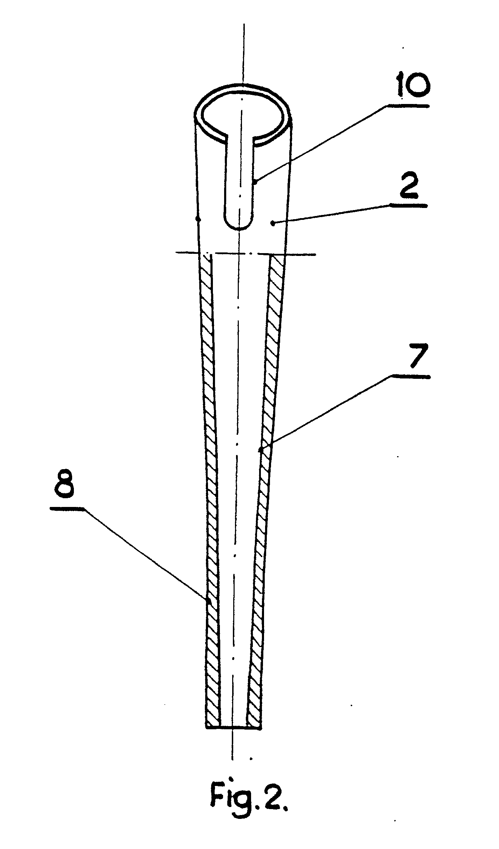

Fig. 2     presents the half section of the joining element.

The hip-bone endoprothesis consists of a housing 1 and a joining element 2. The housing 1 has a brace 3 and a ball 4 mounted on a pivot 5. A wedge-shaped locking lip 6 is located between the brace 3 and the ball 4. The brace 3 has a straight cone shape. The joining element 2 has a cone inner surface 7 and an outer surface 8 composed of a cone and a cylindrical part. At the top, the joining element 2 is cut approximately at the same angle as the thigh-bone 9 and has a lateral orifice matching with the wedge-shaped locking lip.

In order to mount the endoprothesis in the thigh-bone 9 first the bone's head has to be cut off. Then a relevant cavity in the thigh-bone has to be shaped. Further, a cylindrical canal is drilled. Then, the cone surface is obtained with the use of a cone shank cutter matching with the outer profile 8 of the joining element 2. Now, the joining element 2 can be inserted, so that the outer surface 8 adheres firmly to the thigh-bone cavity surface 9. The stem 3 with the ball 4 on the pivot 5 is then introduced into the joining element 2 in the thigh-bone 9, so that the wedge-shaped locking lip 6 enters into the lateral orifice 10 of the joining element 2. As a result, the endoprothesis remains firmly in the thigh-bone cavity 9.

## Claims

1. A hip-joint endoprothesis composed of a housing (1) with a brace (3) and a ball (4) on a pivot (5) as well as of a joining element (2), characterized by providing a straight symmetry axis frustum-shaped brace (3) with a wedge-shaped locking lip (6) below the ball (4), and that the joining element (2) has an inner straight-cut cone surface and a lateral orifice (10) in its upper part, matching with the wedge lip (6).

2. The endoprothesis according to claim 1, characterized by a joining (2) having a cut in its upper part at an angle similar as the thigh-bone.

3. The endoprothesis according to claim 1, characterized by a cylindrical shape of the outer surface (8) of the joining element (2) in its part being the lowest and the most deeply introduced into the bone.

## Revendications

1. Endoprothèse pour l'articulation de la hanche constituée par un logement (1) muni d'une armature (3) et d'une sphère (4) sur un pivot (5) ainsi que d'un élément de liaison (2), caractérisée en ce que l'on prévoit une armature tronconique (3) ayant un axe de symétrie droit avec une lèvre de blocage en forme de coin ou de clavette (6) au-dessous de la sphère (4) et en ce que l'élément de liaison (2) comporte une surface conique intérieure à découpe droite et un orifice latéral (10) dans sa partie supérieure, correspondant à la lèvre en forme de coin ou de clavette (6).

2. Endoprothèse selon la revendication 1, caractérisée par une liaison (2) ayant une découpe dans sa partie supérieure à un angle similaire à celui du fémur.

3. Endoprothèse selon la revendication 1, caractérisée par la forme cylindrique de la surface extérieure (8) de l'élément de liaison (2) dans sa partie la plus basse et la plus profondément introduite dans l'os.

## Patentansprüche

1. Hüftgelenksendoprothese, bestehend aus einem Gehäuse (1) mit einer Klammer (3) und einer Kugel (4) auf einem Drehzapfen (5) sowie aus einem Verbindungselement (2) **gekennzeichnet** durch
Vorsehen einer kegelstumpfförmigen Klammer (3) mit gerader Symmetrieachse und mit einer keilförmigen Verriegelungslippe (6) unterhalb der Kugel (4), und dadurch gekennzeichnet, daß
das Verbindungselement (2) eine innere, gerade geschnittene konische Oberfläche und eine seitliche Öffnung (10) in seinem oberen Teil hat, die mit der Keillippe (6) zusammenpaßt.

2. Endoprothese nach Anspruch 1,
**gekennzeichnet** durch
eine Verbindung (2) mit einem Schnitt in ihrem oberen Teil in einem dem Oberschenkelknochen ähnlichen Winkel.

3. Endoprothese nach Anspruch 1,
dadurch **gekennzeichnet,** daß
der Teil des Verbindungselementes (2) eine zylindrische Form der Außenfläche (8) aufweist, welcher der unterste und am tiefsten in den Knochen eingeführte ist.

Fig.1.

Fig.2.